# EUROPEAN PATENT SPECIFICATION

(11) **EP 3 184 052 B1**
(45) Date of publication and mention of the grant of the patent: **24.07.2019**
(21) Application number: 14903059.5
(22) Date of filing: 01.10.2014
(51) Int. Cl.: A61B 10/02

(54) **CELL COLLECTION PAD FOR EXAMINING CERVICAL DISEASES AND COLLECTION KIT COMPRISING SAME**
PAD ZUR ENTNAHME VON ZELLEN ZUR UNTERSUCHUNG VON ZERVIKALEN ERKRANKUNGEN UND ENTNAHMEKIT DAMIT
TAMPON DE PRÉLÈVEMENT DE CELLULES POUR L'EXAMEN DE MALADIES DU COL DE L'UTÉRUS ET NÉCESSAIRE DE PRÉLÈVEMENT COMPORTANT UN TEL TAMPON

(43) Date of publication of application: 28.06.2017
(73) Proprietor: TCM BIOSCIENCES INC., Gyeonggi-do 13487 (KR)
(72) Inventor: PARK, Young Chul, Seoul 135-958 (KR); JOO, Hee Jae, Yongin-si Gyeonggi-do 448-737 (KR); SHIN, Dong Jin, Seongnam-si Gyeonggi-do 463-956 (KR)
(74) Representative: IPAZ
(86) International application number: PCT/KR2014/009286
(87) International publication number: WO 2016/052780

(56) References cited:
- WO-A1-2004/071304
- KR-A- 20040 074 174
- KR-A- 20060 001 380
- KR-A- 20060 001 380
- KR-A- 20090 033 644
- KR-A- 20110 056 648
- KR-Y1- 200 282 573
- US-A1- 2001 011 167
- US-A1- 2003 023 188

## Description

### [Technical Field]

The present invention relates to a cell collection pad for cervical disease screening and a collection kit including the same. More particularly, the present invention relates to a cell collection pad for cervical disease screening which takes the form of a wearable pad and includes a filter unit collecting uterine cervical cells and an absorption unit absorbing vaginal secretions having passed through the filter unit, thereby collecting uterine cervical cells from the human body in an easy and stable manner, and a collection kit including the same.

### [Background Art]

The uterus is composed of the corpus and the cervix, and uterine cervical cancer refers to a malignant tumor arising from the uterine cervix connected to the vagina. Uterine cervical cancer is the world's second most common type of cancer in women. It is known that about 85% of cervical cancer cases occur in developing countries in Asia, South America, and Africa.

Uterine cervical cancer, one of the most common forms of cancer in women, is a tumor, pathogenesis of which is relatively well known, and is caused by the human papilloma virus. When epithelial cells of the female genitalia are infected with the human papilloma virus during sexual intercourse, DNAs of the human papilloma virus penetrate DNAs in the cell nucleus and proliferate, causing epidermoid carcinoma. Although detection of the infection of the human papilloma virus has been performed by various methods including cervical Pap smear, polymerase chain reaction (PCR) examination is considered the most sensitive and accurate among screening methods for early diagnosis of uterine cervical cancer. PCR examination is sensitive enough to accurately detect uterine cervical cancer only using a single cell. However, for this examination, uterine cervical cells must be collected. For this, a woman needs to go to the hospital and allow a doctor to take cervical cells from her genitalia, which is a burden on her in terms of time and costs as well as causes her to feel embarrassment due to the fact that she reveals her genitals to a doctor.

In addition, a typical instrument for collecting uterine cervical cells is pointed at an end of a cell collection portion thereof and thus can hurt the vaginal wall or the uterine cervix, which can cause bacterial infection, even when a skilled doctor takes uterine cervical cells using the instrument.

Korean Utility Model Application No. 1993-0022216 discloses "Cell collection structure for cervical disease screening". In the aforementioned application, there is proposed an instrument for collecting uterine cervical cells configured to be inserted into the vagina to collect uterine cervical cells.

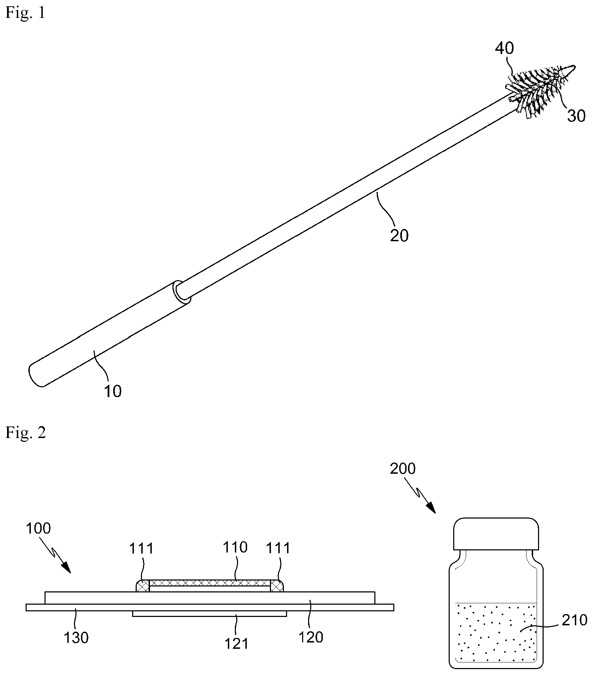

Fig. 1 is a perspective view of an instrument for collecting uterine cervical cells according to the related art. A handle 10 is connected to a cell collection portion 30 through a connection portion 20. The handle 10 has a faceted shape to allow a user to easily collect cervical cells by turning the instrument for collecting uterine cervical cells up and down and right and left while preventing the instrument from slipping out of user's hand during cell collection.

The connection portion 20 has a length allowing the cell collection portion 30 to pass through the vagina to the uterine cervix. The connection portion 30 is provided with a cell collection brush 40 wrapped around the connection portion 30. The cell collection brush 40 is composed of fine strands capable of easily collecting uterine cervical cells. Operation of the instrument for collecting uterine cervical cells according to the related art is as follows. First, a user expands the inside of the vagina using a vaginal speculum. Then, the user holds the handle 10 of the instrument for collecting uterine cervical cells and inserts the instrument into the vagina. Once the cell collection portion 30 of the inserted instrument reaches the uterine cervix, the user turns the handle up and down and right and left such that uterine cervical cells are detached from the uterine cervix and smeared onto the cell collection brush 40. In the aforementioned process, the uterine cervix and the vaginal wall are likely to be wounded and thus can suffer from bacterial infection. In uterine cervix, the user turns the handle up and down and right and left such that uterine cervical cells are detached from the uterine cervix and smeared onto the cell collection brush 40. In the aforementioned process, the uterine cervix and the vaginal wall are likely to be wounded and thus can suffer from bacterial infection. In addition, if the user is not a skilled doctor but a subject, such infection can be more easily effected.

On the other hand, if a subject goes to hospital rather than collecting uterine cervical cells by herself, this is costly and time consuming. Particularly, a subject can be deeply embarrassed that she revealed her genital organs to a doctor. As a result, she will not want to go to hospital and thus cannot detect diseases such as uterine cervical cancer in early stages.

Document WO 2004/071304 A1 discloses an apparatus for collecting cervical cells comprising a filtering unit 10' and an absorbing unit 20. The filtering unit 10' is provided with a pair of cutting lines 14c and 14d at both sides thereof.

Document US 2003/023188 A1 describes a kit for generating a biological sample from a discharge emitted from a portion of a body. The kit includes an absorbent pad for deploying adjacent to the portion of the body and a portable device for preserving and/or pre-processing the sample.

Document KR 2006 0001380 A describes a cervical cells collected pad including a filter unit and a grid-shaped thin film.

Document US 2001/0011167 A1 describes a porous outer sleeve open ended at one longitudinal end thereof and an upper and lower sheets. An outer surface of the lower sheet is provided with an impermeable sheet coated with an adhesive covered, before use of the sanitary napkin, with a removable protective peel-off strip.

### [Disclosure]

### [Technical Problem]

The present invention has been conceived to solve such problems in the art, and it is an aspect of the present invention to provide a cell collection pad for cervical disease screening which allows a subject to collect cervical cells in an easy and safe manner without going to hospital, and a collection kit including the same.

It is another aspect of the present invention to provide a cell collection pad for cervical disease screening which further includes a close contact pad protruding from a surface thereof facing the vaginal opening, thereby increasing the collection rate of cells from the uterine cervix through friction between the close contact pad and the inside of the labia majora, and a collection kit including the same.

It is a further aspect of the present invention to provide a cell collection pad for cervical disease screening which allows a user to easily separate a filter unit and can minimize contamination of cells during removal of the filter unit from an absorption unit by including an improved structure for tearing off and separating the filter unit, and a collection kit including the same.

It is yet another aspect of the present invention to provide a cell collection pad for cervical disease screening which can provide comfortable fit to a wearer in everyday life and can be prevented from being pushed off or folded during strenuous activity while effectively protecting the genitalia and the perineal region, and a collection kit including the same.

### [Technical Solution]

In accordance with one aspect of the present invention, a cell collection pad for cervical disease screening is disclosed according to claim 1. The cell collection pad includes: a filter unit collecting cervical cells; and an absorption unit absorbing vaginal secretions having passed through the filter unit.

Preferably, the filter unit is composed of grids sized to allow vaginal secretions to pass therethrough while preventing cervical cells from passing therethrough.

Preferably, the filter unit is formed of at least one selected from the group consisting of cotton, pulp, and a combination thereof

Preferably, the filter unit includes a super absorbent resin within the grids constituting the filter unit.

Preferably, the super absorbent resin is formed of at least one selected from the group consisting of sodium acrylate, starch/acrylic acid, and zeolite.

Preferably, the filter unit takes the form of a sheet.

filter unit is provided with a separation means allowing the filter unit to be separated from the absorption unit after collection of cervical cells.

separation means is a dotted line which is formed at a boundary between a separation seam of the absorption unit and the filter unit, wherein the dotted line is omitted at at least one point such that the filter unit is folded about the point in the process of separation of the filter unit.

Preferably, the filter unit is formed with an elevated close contact filter at a position corresponding to a valley of the genital area.

Preferably, the absorption unit is formed of at least one selected from the group consisting of cotton, pulp, and a combination thereof.

Preferably, the absorption unit takes the form of a pad.

Preferably, the absorption unit has an adhesive portion to be attached to a fixing member fixing the cell collection pad.

Preferably, the cell collection pad further includes a sheet unit supporting the filter unit and the absorption unit.

Preferably, the sheet unit is formed of a water impermeable material.

Preferably, the sheet unit has at least one central pressing line continuously or discontinuously formed along all or some of a longitudinal central axis of the sheet unit.

In accordance with another aspect of the present invention, a collection kit for cervical disease screening includes: the cell collection pad as set forth above; and a cell fixing container containing a cell fixative for fixing collected cervical cells.

Preferably, the cell fixative is an alcoholic solution or a solution obtained by mixing an alcoholic solution with a solution capable of optimizing a polymerase chain reaction.

Preferably, the solution capable of optimizing a polymerase chain reaction is a PBS buffer solution or paraformaldehyde.

### [Advantageous Effects]

According to the present invention, it is possible to provide a cell collection pad for cervical disease screening which includes a filter unit composed of grids sized to allow vaginal secretions to pass therethrough while preventing cervical cells from passing therethrough and an absorption unit absorbing vaginal secretions having passed through the filter unit, thereby collecting cervical cells in an easy and safe manner, and a collection kit including the same.

Particularly, it is possible to provide a cell collection pad for cervical disease screening which allows a user to collect cervical cells by herself without going to hospital, thereby detecting cervical diseases in early stages, and a collection kit including the same.

In addition, it is possible to provide a cell collection pad for cervical disease screening which further includes a close contact pad protruding from a surface thereof facing the vaginal opening, thereby increasing the collection rate of cells from the uterine cervix through friction between the close contact pad and the inside of the labia majora, and a collection kit including the same.

Further, a cell collection pad for cervical disease screening is provided wherein the cell collection pad allows a user to easily separate a filter unit and minimizes contamination of cells during removal of the filter unit from an absorption unit by including an improved structure for tearing off and separating the filter unit, and a collection kit including the same.

Furthermore, it is possible to provide a cell collection pad for cervical disease screening which can provide comfortable fit to a wearer in everyday life and can be prevented from being pushed off or folded during strenuous activity while effectively protecting the genitalia and the perineal region, and a collection kit including the same.

### [Description of Drawings]

Fig. 1 is a perspective view of an instrument for collecting cervical cells according to the related art.
Fig. 2 shows side views of a cell collection pad for cervical disease and a collection kit including the same.
Fig. 3 is a view illustrating a cervical cell collection process using the cell collection pad for cervical disease screening.
Fig. 4 is a view illustrating a structure and separation process of a filter unit of the cell collection pad for cervical disease screening.
Fig. 5 is a view illustrating a structure and separation process of a filter unit of a cell collection pad for cervical disease screening.
Fig. 6 is a view illustrating a structure and separation process of a filter unit of a cell collection pad for cervical disease screening according to an embodiment of the present invention.
Fig. 7 is a view illustrating a close contact filter and wearing process of the cell collection pad for cervical disease screening according to one embodiment of the present invention.
Fig. 8 is a view of a sheet unit of the cell collection pad for cervical disease screening according to one embodiment of the present invention.

### [Best Mode]

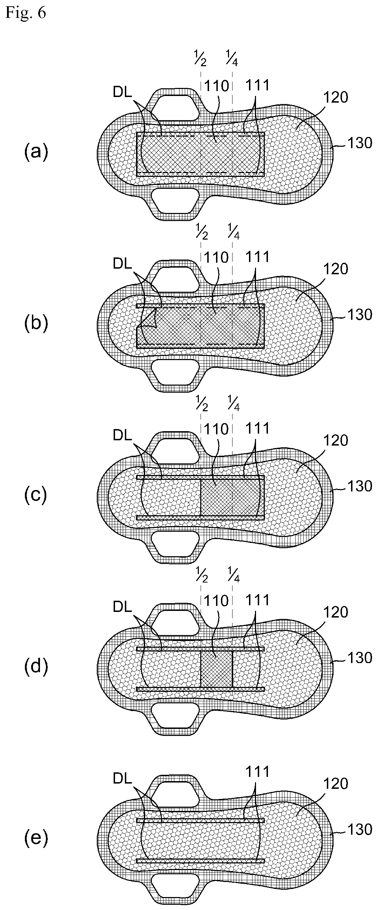

Hereinafter, a cell collection pad for cervical disease screening according to preferred embodiments of the present invention and a collection kit including the same will be described with reference to fig. 6.

Fig. 2 shows side views of a cell collection pad for cervical disease screening not forming part of the present invention and a collection kit including the same.

A cell collection pad 100 according to the present invention may include a filter unit 110 composed of grids sized to allow vaginal secretions to pass therethrough while preventing cervical cells from passing therethrough, and an absorption unit 110 disposed below the filter unit 110 and absorbing vaginal secretions having passed through the filter unit 110. In addition, the cell collection pad may further include a sheet unit 130 configured to support the filter unit 110 and the absorption unit 120 and formed of a water impermeable material to prevent the absorbed vaginal secretions from leaking out.

A collection kit for cervical disease screening includes the cell collection pad and a cell fixing container 210 containing a cell fixative 210 to store the filter unit 110 removed from the cell collection pad 100.

Here, the cell collection pad 100 may include only the filter unit 110 and the absorption unit 120, or may further include the sheet unit 130 depending upon physical conditions of a user and the amount of vaginal secretions.

In Fig. 2, reference numeral 121 is a feature for fixing the cell collection pad 100 and may be an adhesive tape attached to a rear surface of the absorption unit 120 when the cell collection pad is composed of the filter unit 110 and the absorption unit 120 or attached to a rear surface of the sheet unit 130 when the cell collection pad is composed of the filter unit 110, the absorption unit 120, and the sheet unit 130. For example, the adhesive tape may be bonded to an inner surface of a wearer's underpants such that the cell collection pad 100 can remain fixed for a period of time for which cervical cells are collected.

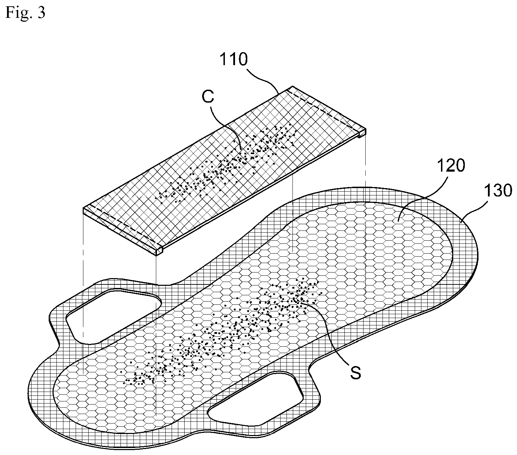

Fig. 3 is a view illustrating a cervical cell collection process using the cell collection pad for cervical disease screening not forming part of the claimed invention.

Referring to Fig. 3, cervical cells C naturally fall out of the uterine cervix, and the separated cervical cells C are mixed with vaginal secretions S and discharged from the vagina. When the discharged cervical cells C and vaginal secretions S reach the filter unit 110, the liquid vaginal secretions composed of fine particles pass through the filter unit 110 and are absorbed by the absorption unit 120, whereas the cervical cells C greater in size than grids of the filter unit 110 are left behind in the filter unit 110 without passing through the filter unit 110. In other words, cervical cells are collected on the filter unit due to size difference between vaginal secretion particles and cervical cells.

Thereafter, the filter unit 110 is removed from the absorption unit 120 for fixation of the collected cervical cells and then put into the cell fixing container 200 containing the cell fixative 210.

Here, the filter unit 110 is composed of grids sized to allow vaginal secretions to pass therethrough while preventing cervical cells from passing therethrough. In other words, the grids are smaller in size than cervical cells and greater in size than vaginal secretion particles.

Since the filter unit 110 is disposed adjacent the vaginal opening, the filter unit is preferably formed of natural fibers harmless to humans, such as cotton, pulp, and a combination thereof.

In addition, the filter unit 100 may also include a super absorbent polymer resin inside the fine grids constituting the filter unit. A super absorbent polymer resin refers to a resin which has a three-dimensional mesh structure obtained by crosslinking between polymer chains or a single chain structure with hydrophilic groups introduced thereinto, thereby exhibiting excellent fluid absorptivity as compared with general polymers. The mesh structure of such a super absorbent polymer has fine channels between chains, and the chains absorb fluid through capillary action. As a result, cervical cells contacting the filter unit 110 can flow into the polymer chains by osmotic pressure. The super absorbent resin may be formed of at least one selected from the group consisting of sodium acrylate, starch/acrylic acid, and zeolite, without being limited thereto.

Since the filter unit 110 is preserved in the cell fixative after collection of cervical cells, the filter unit preferably takes the form of a sheet for easy storage.

The absorption unit 120 absorbing vaginal secretions having passed through the filter unit 110 is preferably formed of a highly absorbent material in order to effectively absorb the vaginal secretions having passed through the filter unit 110. For example, the absorption unit may be formed of cotton, pulp, or a combination thereof. Particularly, the absorption unit may be formed of pulp and a non-woven fabric (Air-laid) having a sufficient space to absorb and store vaginal secretions. In addition, it is desirable that the absorption unit 120 take the form of a pad that is thinner than commercially available female pads, considering that the amount of vaginal secretions is smaller than that of menstrual fluids.

The cell collection pad may further include the sheet unit 130 disposed on a rear side of the filter unit 110 and the absorption unit 120 depending upon physical conditions of a user and the amount of vaginal secretions. The sheet unit 130 is formed of a water impermeable material which allows moisture to be discharged therethrough, but does not allow water to be discharged therethrough to prevent vaginal secretions from leaking out. As a material having this feature, a cover made of a breathable polyethylene film may be used. The sheet unit 130 has a size greater than or equal to that of the absorption unit 120 and is fused to the absorption unit 110 at an edge thereof.

Preferably, the cell fixative 210 is an alcoholic solution or a solution obtained by mixing an alcoholic solution with a solution capable of optimizing a polymerase chain reaction. Examples of the alcoholic solution include ethanol, methanol, and the like, and examples of the solution capable of optimizing a polymerase chain reaction include a PBS buffer solution, paraformaldehyde, and the like.

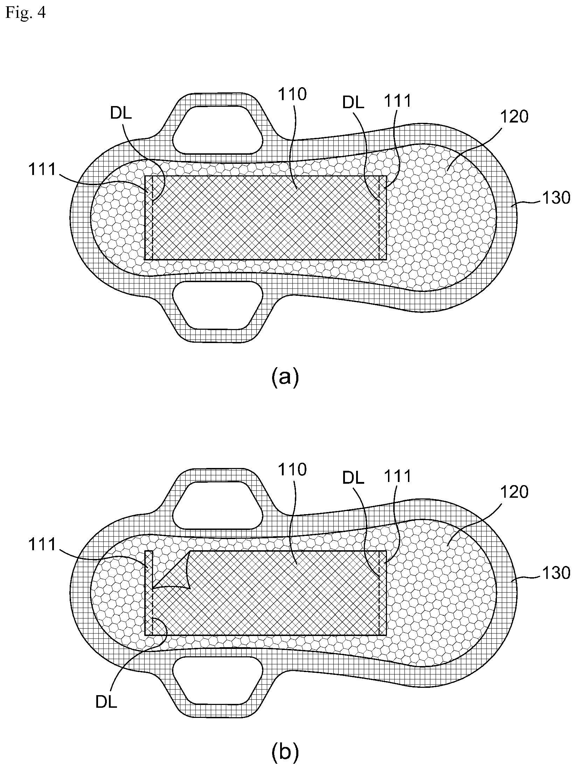

Fig. 4 is a view illustrating a structure and separation process of the filter unit of the cell collection pad for cervical disease screening not forming part of the claimed invention.

In Fig. 4, the cell collection pad 100 is shown as further including the sheet unit 130 in addition to the filter unit 110 and the absorption unit 120.

Referring to Fig. 4, the filter unit 110 is attached to an upper side of the absorption unit 120 through separation seams 111 respectively provided to both ends of the filter unit 110. The separation seams 111 are interposed between the filter unit 110 and the absorption unit 120 at both ends of the filter unit 110. Since the separation seams 111 have a predetermined thickness and the filter unit is attached to the absorption unit through the separation seams 111, the filter unit 110 can be easily separated from the absorption unit 120 after collection of cervical cells. In other words, since the separation seam 111 is thicker than the sheet type filter unit 110, the separation seam 111 is not easily bent or torn by virtue of stiffness thereof even when receiving a force during separation of the filter unit 110 from the absorption unit 120. As a result, it is possible to prevent cervical cells on the filter unit 110 from being ruptured or damaged during the separation process.

Thus, when a user pulls the filter unit 110 along a dotted line DL, as shown in Fig. 4(a), the filter unit 110 is torn off from the right and left separation seams 111, such that the filter unit 110 is naturally separated from the absorption unit, as shown in Fig. 4(b).

Such a separation process of the filter unit 110 is easily and quickly achieved through the separation seams 111 and the dotted line DL.

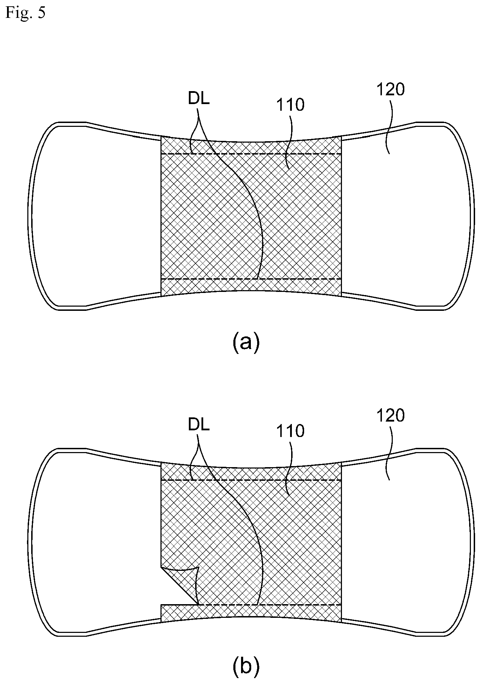

Fig. 5 is a view illustrating a structure and separation process of a filter unit of a cell collection pad for cervical disease screening not forming part of the invention as claimed.

In Fig. 5, a cell collection pad 100 is shown as being composed only of a filter unit 110 and an absorption unit 120.

Referring to Fig. 5, the filter unit 110 is attached to an upper side of the absorption unit 120. Here, the filter unit 110 is attached to the absorption unit 120 to be wrapped around a front surface and rear surface (not shown) of the absorption unit 120. The filter unit 110 may be attached to all or some of the rear surface of the absorption unit 120.

Since the filter unit 110 is wrapped around the absorption unit 120, the filter unit 110 must be provided with a separation means for separation of the filter unit 110 from the absorption unit 120 after collection of cervical cells. As shown in Fig. 5, the filter unit 110 is provided with two dotted lines DL which are formed on the absorption unit 120 and are parallel to a longitudinal direction of the absorption unit 120.

Thus, when a user pulls the filter unit 110 along the dotted lines DL, as shown in Fig. 5(a), the filter unit 110 is torn off between the dotted lines, such that the filter unit 110 is easily and naturally separated from the absorption unit, as shown in Fig. 5(b).

Fig. 6 is a view illustrating a structure and separation process of a filter unit of a cell collection pad for cervical disease screening according to an embodiment of the present invention.

In Fig. 6, a cell collection pad 100 is shown as further including a sheet unit 130 in addition to a filter unit 110 and an absorption unit 120.

Referring to Fig. 6, the filter unit 110 is attached to an upper side of the absorption unit 120 through separation seams 111 respectively provided on both sides of the filter unit in a longitudinal direction of the filter unit 110. In this embodiment, the filter unit 110 is naturally folded in quarter in the process of being separated from the absorption unit 120.

Dotted lines DL are respectively formed at both boundaries between the filter unit 110 and the separation seams 120. As shown in Fig. 6(a), the dotted lines DL are omitted at the 1/2 point of the overall length of the filter unit 110 and at the 1/4 point located on one side of the 1/2 point. Such a configuration in which the dotted lines DL are omitted in this way causes a process of separating the filter unit to be temporally interrupted at the points at which the dotted lines DL are omitted, when a user removes the filter unit 100 while applying a force only sufficient to tear off the dotted lines DL.

Thus, when a user pinches an end of the filter unit 110 and slowly pulls the end, as shown in Fig. 6(b), the filter unit 110 is separated along the dotted lines DL and easily removed to the 1/2 point of the overall length of the filter unit 110 at which the dotted lines DL are omitted, as shown in Fig. 6(c). As a result, the filter unit 110 is naturally folded in half. Then, when the user pinches the other end of the filter unit 110 and slowly pulls the end, as shown in Fig. 6(b), the filter unit 110 is removed to the 1/4 point of the overall length of the filter unit 110 at which the dotted lines DL are omitted. As a result, the filter unit 110 is naturally folded in quarter. Finally, the user may completely remove the quarter folded filter unit 110 using the dotted lines DL, as shown in Fig. 6(e).

Actually, the filter unit 110 is relatively long and is thus difficult to remove all at once even when dotted lines are provided. As a result, since the filter unit is touched by a user here and there while a user tries to remove the filter unit several times, collected cervical cells are likely to be contaminated by the human body. In addition, when such a long filter unit 110 is pushed all at once into a cell fixing container 200 having a narrow opening, airborne contamination and contact contamination can be effected.

In this embodiment, since the filter unit 110 is folded in quarter in the process of separation of the filter unit 110, it is possible to prevent contamination by user's hand and to prevent airborne contamination and contact contamination when the filter unit is put into the cell fixing container 200.

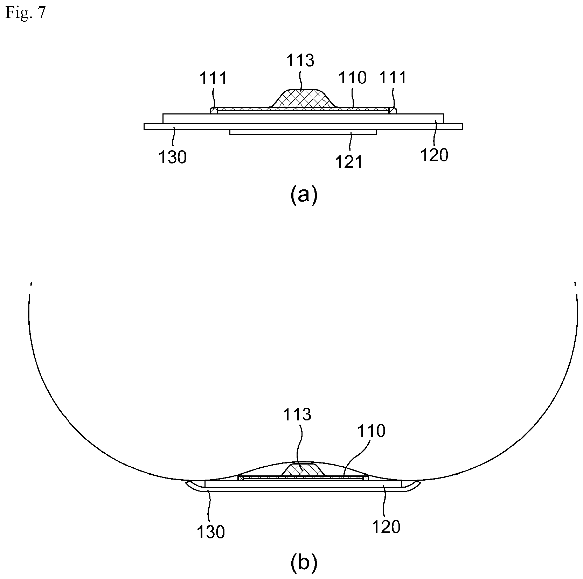

Fig. 7 is a view illustrating a close contact filter and wearing process of the cell collection pad for cervical disease screening according to one embodiment of the present invention.

Referring to Fig. 7(a), in the cell collection pad 100, the filter unit 110 collecting cervical cells may be partially elevated a predetermined height to form a close contact filter 113, which is pressed against a valley of the genitals during wearing of the cell collection pad 100.

The close contact filter 113 may be formed of the same material as the filter unit 110. As described above, the close contact filter 113 is formed by partially elevating a surface of the filter unit 110 by a predetermined height. Thus, as shown in Fig. 7(b), the close contact filter 113 is pressed against a valley of the genitals during wearing of the cell collection pad 100 without leaving a space between the valley and the close contact filter 113, whereby secreted cervical cells can be stably absorbed through the close contact filter 113 even when a user is lying down to sleep.

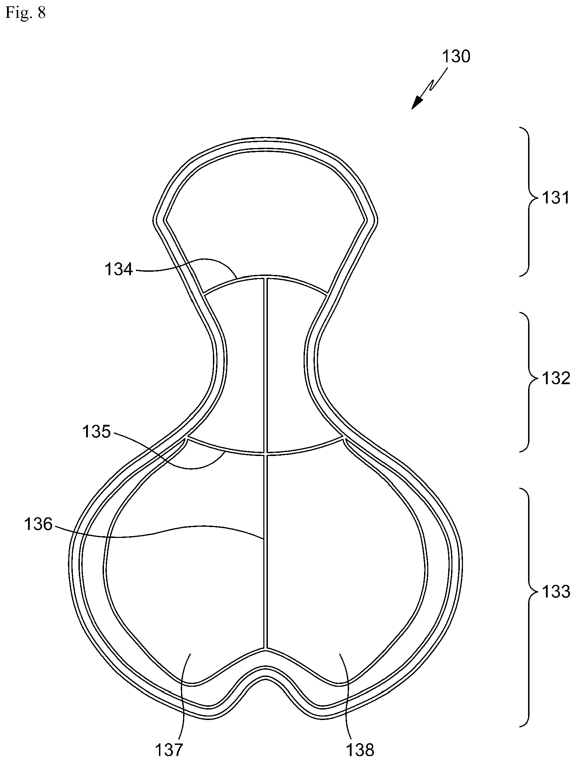

Fig. 8 is a view of the sheet unit of the cell collection pad for cervical disease screening according to one embodiment of the present invention.

The sheet unit 130 supports the filter unit 110 and the absorption unit 120 and is formed of an impermeable material to prevent absorbed vaginal secretions from leaking out. In addition, the sheet unit is required to provide comfortable and stable fit during movement of the human body in that the cell collection pad 100 needs to be worn for a relatively long time.

For this, as shown in Fig. 8, the sheet unit 130 may be composed of a front portion 131, a connection portion 132, and a rear portion 133. Here, the absorption unit 120 may be attached across the front portion 131 and the connection portion 132, across the connection portion 132 and the rear portion 133, or across the front portion 131, the connection portion 132, and the rear portion 133.

The rear portion 133 is a portion on which the pelvis is seated, and has a greater area than the front portion 131 to accommodate the right pelvis and the left pelvis. The front portion 131 is a portion contacting a front side of the perineal region, and connection portion 132 connecting the front portion 131 to the rear portion 133 contacts the perineal region and forms a slender waist.

Here, the sheet unit 130 is formed with a front boundary pressing line 134 traversing the boundary between the front portion 131 and the connection portion 132 and a rear boundary pressing line 135 traversing the boundary between the rear portion 133 and the connection portion 132. Each of the front boundary pressing line 134 and the rear boundary pressing line 135 may be formed continuously, as shown in Fig. 8, or may be formed discontinuously. The pressing lines may be formed continuously or discontinuously depending upon the desired shape maintenance properties of the sheet unit 130 and desired wrinkle flexibility of the sheet unit 130. In addition, it is desirable that at least one pressing line 134, 135 be formed so long as a portion of the pressing line contacting the human body is comfortable.

When the sheet unit 130 is formed with the pressing lines 134, 135, the sheet unit 130 is prevented from being pushed off or folded since the pressing lines 134, 135 can absorb a force that is generated by friction during movement of the human body causing the sheet unit to be pushed off. As a result, the sheet unit can provide a comfortable fit to a user.

The direction of a force causing the sheet unit to be pushed off or folded, resulting from walking or running motion, mainly lies on a line of action connecting the center of the connection portion 132 to the outer periphery of the sheet unit 130. In order to effectively block such an external force, it is desirable that the pressing lines be formed in a direction perpendicular to the direction of the external force. More preferably, each of the front boundary pressing line 134 and the rear boundary pressing line 135 has a curved shape having a curvature toward the center of the sheet unit to effectively prevent the sheet unit 130 from being pushed off or folded. In other words, it is desirable that the front boundary pressing line 134 form a curve that is convex in a direction of the front portion 131, and the rear boundary pressing line 135 form a curve that is convex in a direction of the rear portion 133. The reason why the pressing lines 134, 135 of the sheet unit 130 are formed at the boundary between the connection portion 132 and the front portion 131 and the boundary between the connection portion 132 and the rear portion 133 is because the external force causing the sheet unit 130 to be pushed off is concentrated at the boundary regions.

As shown in the drawings, the sheet unit according to the present invention may further be formed with a central pressing line in a longitudinal direction of the sheet unit 130. In addition, the central pressing line may include at least one central pressing line.

As above, the present invention has been described with reference to preferred embodiments in conjunction with the accompanying drawings. Although specific terms have been used herein, it should be understood that these terms are used for illustration only and are not intended to limit the scope of the present invention. Thus, it should be understood that various modifications, variations, and alterations can be made by those skilled in the art without departing from the scope of the present invention. Therefore, the scope of the invention should be limited only by the accompanying claims.

## Claims

1. A cell collection pad (100) for cervical disease screening, comprising:
a filter unit (110) for collecting cervical cells; and
an absorption unit (120) for absorbing vaginal secretions having passed through the filter unit,
wherein the filter unit (110) is provided with a separation means allowing the filter unit to be separated from the absorption unit (120) after collection of cervical cells,
**characterized in that** the separation means is a dotted line (DL), the dotted line being formed at a boundary between a separation seam (111) of the absorption unit (120) and the filter unit (110), and wherein the dotted line (DL) is omitted at at least one point such that the filter unit (110) is folded about the point in the process of separation of the filter unit.

2. The cell collection pad according to claim 1, wherein the filter unit (110) is composed of grids sized to allow vaginal secretions to pass therethrough while preventing cervical cells from passing therethrough.

3. The cell collection pad according to claim 2, wherein the filter unit (110) is formed of at least one selected from the group consisting of cotton, pulp, and a combination thereof.

4. The cell collection pad according to claim 2, wherein the filter unit (110) comprises a super absorbent resin within the grids constituting the filter unit.

5. The cell collection pad according to claim 4, wherein the super absorbent resin is formed of at least one selected from the group consisting of sodium acrylate, starch/acrylic acid, and zeolite.

6. The cell collection pad according to claim 1, wherein the filter unit (110) takes the form of a sheet.

7. The cell collection pad according to claim 1, wherein the filter unit (110) is formed with an elevated close contact filter (113) at a position corresponding to a valley of the genital area.

8. The cell collection pad according to claim 1, wherein the absorption unit (120) is formed of at least one selected from the group consisting of cotton, pulp, and a combination thereof.

9. The cell collection pad according to claim 1, wherein the absorption unit (120) takes the form of a pad.

10. The cell collection pad according to claim 1, wherein the absorption unit (120) has an adhesive portion to be attached to a fixing member fixing the cell collection pad.

11. The cell collection pad according to claim 1, further comprising:
a sheet unit (130) supporting the filter unit (110) and the absorption unit (120).

12. The cell collection pad according to claim 11, wherein the sheet unit (130) is formed of a water impermeable material.

13. The cell collection pad according to claim 11, wherein the sheet unit (130) has at least one central pressing line continuously or discontinuously formed along all or some of a longitudinal central axis of the sheet unit.

14. A collection kit for cervical disease screening, comprising:
the cell collection pad according to any one of claims 1 to 13; and
a cell fixing container containing a cell fixative for fixing collected cervical cells.

15. The cell collection kit according to claim 14, wherein the cell fixative is an alcoholic solution or a solution obtained by mixing an alcoholic solution with a solution capable of optimizing a polymerase chain reaction.

16. The cell collection kit according to claim 15, wherein the solution capable of optimizing a polymerase chain reaction is a PBS buffer solution or paraformaldehyde.

## Patentansprüche

1. Pad (100) zur Entnahme von Zellen für die Erkennung zervikaler Erkrankungen, mit:
einer Filtereinheit (110), zum Entnehmen zervikaler Zellen; und einer Absorptionseinheit (120) zum Absorbieren vaginaler Sekrete, die die Filtereinheit passiert haben,
wobei die Filtereinheit (110) ein Trennelement aufweist, das es gestattet, die Filtereinheit nach dem Entnehmen der zervikalen Zellen von der Absorptionseinheit (120) zu trennen,
**dadurch gekennzeichnet, dass** das Trennelement eine punktierte Linie (DL) ist, die an der Grenze zwischen einer Trennnaht (111) der Absorptionseinheit (120) und der Filtereinheit (110) ausgebildet ist, und wobei die punktierte Linie (DL) an zumindest einem Punkt ausgespart ist, sodass die Filtereinheit (110) beim Vorgang des Trennens der Filtereinheit über den Punkt gefaltet wird.

2. Pad zur Entnahme von Zellen nach Anspruch 1, wobei die Filtereinheit (110) aus Gittern aufgebaut ist, die derart dimensioniert sind, dass sie ein Hindurchtreten vaginaler Sekrete gestatten, während zervikale Zellen am Hindurchtreten gehindert werden.

3. Pad zur Entnahme von Zellen nach Anspruch 2, wobei die Filtereinheit (110) aus zumindest einem Material, das ausgewählt ist aus der Gruppe bestehend aus Baumwolle, Zellstoff oder Kombinationen davon, ausgebildet ist.

4. Pad zur Entnahme von Zellen nach Anspruch 2, wobei die Filtereinheit (110) ein super-absorbierendes Harz, das innerhalb der Gitter, die die Filtereinheit ausbilden, angeordnet ist, aufweist.

5. Pad zur Entnahme von Zellen nach Anspruch 4, wobei das super-absorbierende Harz aus zumindest einem Material, das ausgewählt ist aus der Gruppe bestehend aus Natriumacrylat, Stärke/Acrylsäure und Zeolith, ausgebildet ist.

6. Pad zur Entnahme von Zellen nach Anspruch 1, wobei die Filtereinheit (110) die Form einer Folie aufweist.

7. Pad zur Entnahme von Zellen nach Anspruch 1, wobei die Filtereinheit (110) mit einem erhabenen Nahkontakt-Filter (113) in einer Position, die mit einem Tal des Genitalbereichs korrespondiert, ausgebildet ist.

8. Pad zur Entnahme von Zellen nach Anspruch 1, wobei die Absorptionseinheit (120) aus zumindest einem Material, das ausgewählt ist aus der Gruppe bestehend aus Baumwolle, Zellstoff oder Kombinationen davon, ausgebildet ist.

9. Pad zur Entnahme von Zellen nach Anspruch 1, wobei die Absorptionseinheit (120) die Form eines Pads aufweist.

10. Pad zur Entnahme von Zellen nach Anspruch 1, wobei die Absorptionseinheit (120) einen Klebeabschnitt zum Befestigen an einem Fixierelement, welches das Pad zur Entnahme von Zellen fixiert, aufweist.

11. Pad zur Entnahme von Zellen nach Anspruch 1, das zudem eine Folieneinheit (130) aufweist, welche die Filtereinheit (110) und die Absorptionseinheit (120) verstärkt.

12. Pad zur Entnahme von Zellen nach Anspruch 11, wobei die Folieneinheit (130) aus einem wasserundurchlässigen Material ausgebildet ist.

13. Pad zur Entnahme von Zellen nach Anspruch 11, wobei die Folieneinheit (130) zumindest eine zentrale Drucklinie aufweist, die kontinuierlich oder diskontinuierlich entlang einer gesamten oder eines Teils einer Längs-Zentralachse der Folieneinheit ausgebildet ist.

14. Entnahme-Kit für die Erkennung zervikaler Erkrankungen, mit:
dem Pad zur Entnahme von Zellen nach einem der Ansprüche 1 bis 13 und
einem Zellfixierbehälter, der ein Fixiermittel zum Fixieren der gesammelten Zellen beinhaltet.

15. Entnahme-Kit nach Anspruch 14, wobei das Fixiermittel eine alkoholische Lösung oder eine Lösung ist, die durch Mischen einer alkoholischen Lösung mit einer Lösung, die geeignet ist eine Polymerase-Kettenreaktion zu optimieren, erhalten wird.

16. Entnahme-Kit nach Anspruch 15, wobei die zum Optimieren einer Polymerase-Kettenreaktion geeignete Lösung eine PBS-Pufferlösung oder Paraformaldehyd ist.

## Revendications

1. Tampon de prélèvement de cellules (100) pour l'examen de maladies du col de l'utérus, comprenant :
une unité de filtrage (110) servant à prélever des cellules cervicales ; et
une unité d'absorption (120) servant à absorber les sécrétions vaginales ayant traversé l'unité de filtrage,
dans lequel l'unité de filtrage (110) est pourvue d'un moyen de séparation permettant de séparer l'unité de filtrage de l'unité d'absorption (120) après le prélèvement des cellules cervicales,
**caractérisé en ce que** le moyen de séparation est une ligne pointillée (DL), la ligne pointillée étant formée au niveau d'une frontière entre une couture de séparation (111) de l'unité d'absorption (120) et de l'unité de filtrage (110), et dans lequel la ligne pointillée (DL) est omise au niveau d'au moins un point de telle sorte que l'unité de filtrage (110) puisse être pliée autour du point durant le processus de séparation de l'unité de filtrage.

2. Tampon de prélèvement de cellules selon la revendication 1, dans lequel l'unité de filtrage (110) est composée de grilles dimensionnées pour permettre le passage des sécrétions vaginales mais empêcher le passage des cellules cervicales.

3. Tampon de prélèvement de cellules selon la revendication 2, dans lequel l'unité de filtrage (110) est formée d'au moins un matériau sélectionné dans le groupe consistant en coton, cellulose et une combinaison de ceux-ci.

4. Tampon de prélèvement de cellules selon la revendication 2, dans lequel l'unité de filtrage (110) comprend une résine super-absorbante à l'intérieur des grilles constituant l'unité de filtrage.

5. Tampon de prélèvement de cellules selon la revendication 4, dans lequel la résine super-absorbante est formée d'au moins un élément sélectionné dans le groupe consistant en acrylate de sodium, amidon/acide acrylique et zéolithe.

6. Tampon de prélèvement de cellules selon la revendication 1, dans lequel l'unité de filtrage (110) se présente sous forme de feuille.

7. Tampon de prélèvement de cellules selon la revendication 1, dans lequel l'unité de filtrage (110) est formée avec un filtre élevé à contact intime (113) à une position correspondant à une vallée de la zone génitale.

8. Tampon de prélèvement de cellules selon la revendication 1, dans lequel l'unité d'absorption (120) est formée d'au moins un matériau sélectionné dans le groupe consistant en coton, cellulose, et une combinaison de ceux-ci.

9. Tampon de prélèvement de cellules selon la revendication 1, dans lequel l'unité d'absorption (120) se présente sous forme de tampon.

10. Tampon de prélèvement de cellules selon la revendication 1, dans lequel l'unité d'absorption (120) présente une partie adhésive destinée à être attachée à un élément de fixation qui fixe le tampon de prélèvement de cellules.

11. Tampon de prélèvement de cellules selon la revendication 1, comprenant en outre :
une unité de feuille (130) qui supporte l'unité de filtrage (110) et l'unité d'absorption (120).

12. Tampon de prélèvement de cellules selon la revendication 11, dans lequel l'unité de feuille (130) est formée dans un matériau imperméable à l'eau.

13. Tampon de prélèvement de cellules selon la revendication 11, dans lequel l'unité de feuille (130) présente au moins une ligne de pression centrale formée de manière continue ou discontinue le long de la totalité ou d'une partie d'un axe central longitudinal de l'unité de feuille.

14. Kit de prélèvement pour l'examen de maladies du col de l'utérus, comprenant :
le tampon de prélèvement de cellules selon l'une quelconque des revendications 1 à 13 ; et
un récipient de fixation de cellules contenant un fixateur de cellules pour fixer les cellules cervicales prélevées.

15. Kit de prélèvement de cellules selon la revendication 14, dans lequel le fixateur de cellules est une solution alcoolique ou une solution obtenue en mélangeant une solution alcoolique avec une solution capable d'optimiser une réaction en chaîne par polymérase.

16. Kit de prélèvement de cellules selon la revendication 15, dans lequel la solution capable d'optimiser une réaction en chaîne par polymérase est une solution tampon PBS ou du paraformaldéhyde.
